# EUROPEAN PATENT SPECIFICATION

(11) **EP 2 330 100 B1**
(45) Date of publication and mention of the grant of the patent: **11.01.2017**
(21) Application number: 10014181.1
(22) Date of filing: 02.11.2010
(51) Int. Cl.: C07D 305/14, A61K 39/395, A61P 35/00, A61P 43/00

(54) **An improved process for preparation of taxane derivatives**
Verbessertes Verfahren zur Herstellung von Taxanderivaten
Procédé amélioré pour la préparation des dérivés de taxane

(30) Priority: 04.11.2009 IN MU25592009
(43) Date of publication of application: 08.06.2011
(73) Proprietor: Emcure Pharmaceuticals Limited, Maharashtra 411026 (IN)
(72) Inventor: Gurjar, Mukund, Keshav, Pune-411 026 (IN); Sonawane, Swapnil, Panditrao, Pune-411 026 (IN); Patil, Pankaj, Shallkrao, Pune-411 026 (IN); Mehta, Samit, Satish, Pune-411 026 (IN)
(74) Representative: Docherty, Robert Charles

(56) References cited:
- WO-A1-2006/009518
- US-A- 5 856 532
- SON, JOO-SUN; CHOI, HYUNG-KYOON; HONG, SEUNG-SUH: "Recovery of extracellular paclitaxel from suspension culture of Taxus chinensis by adding inorganic salts", BIOTECHNOLOGY LETTERS, vol. 23, no. 9, 2001, pages 723-725, XP002631341, ISSN: 0141-5492, DOI: 10.1023/A:1010350902765

## Description

### FIELD OF THE INVENTION

The present invention relates to an improved synthetic method for preparation of taxane derivatives. More particularly, this invention relates to a novel method for minimizing the levels of the principal degradation product, namely the epi-isomer at the 7-position of baccatin ring formed in taxane derivative during synthesis and / or storage of docetaxel.

### BACKGROUND AND PRIOR ART OF THE INVENTION

The chemical compounds, which are generically known as paclitaxel (I) and docetaxel (II), because of their novel molecular architecture, potent biological activity and fascinating mode of action have become the mainstay in cancer chemotherapy. Currently, paclitaxel, which is marketed under the brand name of Taxol, is approved for the treatment of breast cancer, ovarian cancer either as a first-line or second-line therapy, as a first-line treatment for non-small cell lung cancer, and also for treatment of advanced AIDS-related Kaposi's sarcoma. Similarly, docetaxel is also approved for treatment of locally advanced or metastatic breast cancer, either alone or in combination with cisplatin for the treatment of patients with unresectable, locally advanced or metastatic non-small cell lung cancer, in combination with prednisone for patients with androgen independent (hormone refractory) metastatic prostate cancer, in combination with doxorubicin and cyclophosphamide for the adjuvant treatment of patients with operable nodepositive breast cancer, in combination with cisplatin and fluorouracil for the treatment of locally advanced squamous cell carcinoma of the head and neck.

| | **R'** | **R** |
|---|---|---|
| **Paclitaxel (I)** | Ac | -C(O)-Ph |
| **Docetaxel (II)** | H | -C(O)-OBu-t |
| 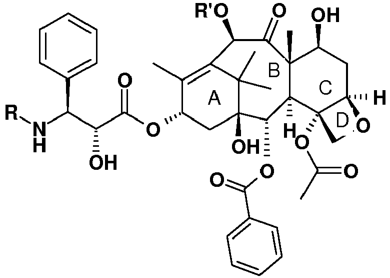 | | |

Paclitaxel is found in several species of yew (genus Taxus, family Taxaceae) trees; however, the concentration of this compound is very low. Paclitaxel, docetaxel and their analogues such as cabazitaxel are built upon the baccatin III framework. Since, the safety and efficacy of paclitaxel and docetaxel are now proven; these compounds continue to stimulate synthetic chemists for further refinement of chemical development process. Further, these taxane derivatives present a plethora of potential problems related to synthesis and pharmaceutical composition primarily due to stability problems. Therefore, a large number of reports are available in the arena of paclitaxel and docetaxel synthesis.

Docetaxel is marketed worldwide in its trihydrate form and which was first disclosed in US 6,197,980 by Durand et. al. Subsequently, in US 6,022,985, Authelin et al disclosed that the trihydrate form has a substantially greater stability than that of anhydrous form. The document further discloses that trihydrate docetaxel is stable at 4°C, 25°C and 35°C in an atmosphere with 90% relative humidity upto 18 months without any danger to its hydrated form whereas anhydrous form has a tendency to slowly change to the trihydrate form. Further the document also discloses that it may be advantageous to perform the crystallization in the presence of an acid such as ascorbic acid, however, there is no data in support of any advantageous effect obtained by addition of acid during crystallization.

Recently, due to enhanced stability of trihydrate form of taxane derivative, there has been an increasing interest in the methods for preparation of taxane, especially docetaxel in trihydrate form.

Sharma et al in US 6,838,569 discloses a process for preparation of docetaxel and paclitaxel trihydrate using acetonitrile and water. However, again there is no mention of any stability data of docetaxel obtained by such process. Similarly, Li et al in US 7,332,617, also discloses that docetaxel trihydrate could be prepared by using acetone/water combination.

US 5,856,532 A relates to a process for the isolation of xylosides of taxol A,B and C (taxol analogues A, B, C) from the stem bark of the Himalayan yew *Taxus wallichiana* and conversion of these isolated taxol analogues 7-xylosyl-10-deacetyl taxols A,B and C into the intermediate product 10-deacetyl taxols A, B, C of formula (2). Herein, after few initial chemical reactions for preparation of Taxol A, B & C, an organic solvent is employed for extraction of the taxane derivatives which, after aqueous washings, is passed through a metal salt like sodium sulphate and then concentrated. The primary objective of the metal salt is as a drying agent, which removes residual water from the organic phase before proceeding for concentarting the organic phase to obtain a residue. This is then purified by chromatography by using a silica gel column.

WO 2006/009518 discloses a method for the production of paclitaxel by using the strain CGMCC No. 0899, comprising incubation of the microorganism in a medium so as to produce and concentrated paclitaxel in the cells of the micro-organism and/or the medium and involves isolation of the taxane derivative by collecting and/or purifying the paclitaxel from the cells and/or medium. Organic sources such as glucose, sucrose, maltose, fructose, glycerol, starch, lactose and / or galactose or non-organic materials (phosphate, magnesium salts such as magnesium sulphate, ferric salts such as ferrous sulphate, ferric chloride; sodium salts, such as sodium potassium tartarate) are added to the medium for accelerating the microbe's growth and for increasing the rate of paclitaxel (taxane) production.

The culture was incubated at 25°C for 16 days and filtered to get wet mycelia, which was then dried in the oven at 60°C. The dried mycelia was then continuously extracted with an organic solvent such as chloroform for 12 hours and the organic layer was then lyophilized to get a sample, which was purified by chromatography. Thus the reaction milieu of the preparation of paclitaxel is a biological system involving microbe wherein inorganic metal salts used herein as microbial growth enhancer.

Biotechnology Letters (2001) vol-23(9), page 723-725 describes a method for recovering partially purified paclitaxel from extracellular culture medium without using organic solvent. The method comprises of adding a 7.8mM solution of MgSO₄ to a culture medium containing baccatin III and paclitaxel whereby paclitaxel separated out selectively from the culture medium of the Taxus species. The culture medium contains B5 modified medium which comprises several metal salt. The role of the metal salt is basically to separate out the taxane derivative from the medium and in doing so a very high weight ratio of salt to paclitaxael is to be maintained.

However, it should be noted that anhydrous as well as relatively greater stable trihydrate form of taxane are liable to undergo degradation, at times drastic, under various manufacturing and storage conditions, for example temperature, acidic and alkaline media, light etc. One of the probable and possibly the most prone pathway for degradation normally observed is epimerization of the hydroxyl group at position 7, which results in formation of 7-epi-docetaxel by way of a retro aldol reaction. The epimerization reaction has been observed in alkaline, neutral as well as strongly acidic media. Further, in acidic media or in presence of electrophilic agents, opening and/or rearrangements of D ring as well as ring B ring is prominent, whereas in basic media cleavage of the ester groups at positions 2, 4 and / or 13 is observed. It is also reported that 10-deacetyl baccatin III, which is normally employed as a starting material for synthesis of docetaxel, and its epimer viz., 7-epi-10-deacetyl baccatin III are formed in basic conditions. Basic conditions also lead to formation of 7-epi-10-oxo-10-deacetyl baccatin III. Of these major degradation products of docetaxel, the formation of 7-epi-docetaxel by way of a retro aldol reaction has vexed researchers in this field because 7-epi-docetaxel is thermodynamically more stable and hence formation is favored. The probable mechanism for formation of 7-epimer of taxane derivative is summarized in Scheme -1.

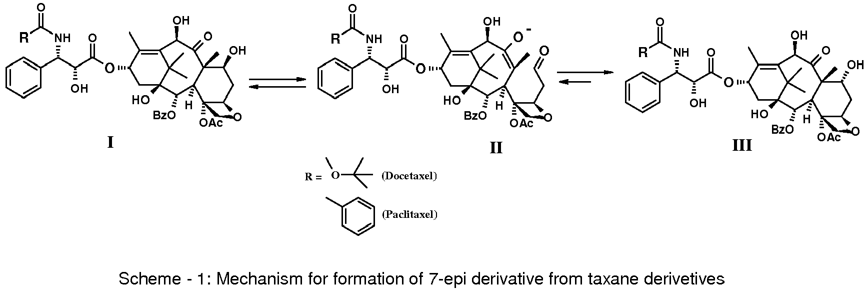

The degradation of taxane derivatives has been a matter of concern as the degradation can result in products with reduced desired pharmacological activity or at times may result in completely inactive products or products with completely different pharmacological and toxicological pattern. Moreover, health authorities all over the world have very stringent norms for permissible limits of these degradation products in the final formulation.

Hence, there is a need for a synthetic process, which will minimize the formation of impurities during reaction as well as on storage of docetaxel.

### OBJECTS OF THE INVENTION

An object of the present invention is to provide a taxane derivative which has minimum impurities resulting from degradation.

Another object of the present invention is to provide a taxane derivative, which is more stable for pharmaceutically acceptable duration time.

Still another object of the present invention is to provide a process for minimization of impurities formed in a taxane derivative during synthesis or on storage.

A further object of the present invention is to provide a pharmaceutical composition prepared by using a taxane derivative with minimum degradation impurities.

Yet further another object of the present invention is to provide a method to reduce the formation of thermodynamically stable epimerization product i.e. 7-epi-docetaxel or 7-epi-paclitaxel formed in docetaxel and paclitaxel respectively.

### SUMMARY OF THE INVENTION

According to an aspect of the invention there is provided a process for the preparation of taxane derivatives comprising the steps:
i) forming a reaction mixture of a taxane derivative and a water soluble metal salt selected from the group consisting of aluminum sulfate, potassium aluminum sulfate, lithium biphosphate, sodium dihydrogen phosphate, potassium trisulfite, potassium hydrogen sulfate, potassium dihydrogen phosphate, sodium hydrogen sulfite, calcium aluminium sulfate wherein said salt is provided in a concentration of 1 to 10% by weight of the taxane derivative; and
ii) isolating the taxane derivative from the reaction mixture in the presence of the metal salt wherein the degradation product 7 epimer is reduced to less than 0.2% of the isolated taxane derivative.

In a preferred method of the invention said metal salt is potassium aluminum sulfate.

In a preferred method of the invention the said metal salt is added in concentration of up to 2-3% by weight of taxane derivative.

In a further preferred method of the invention said 7 epimer is 7-epi-docetaxel or 7-epi-paclitaxel.

According to a further aspect of the invention there is provided Docetaxel isolated in presence of 1-10% of a water soluble metal salt selected from the group consisting of aluminum sulfate, potassium aluminum sulfate, lithium biphosphate, sodium dihydrogen phosphate, potassium trisulfite, potassium hydrogen sulfate, potassium dihydrogen phosphate, sodium hydrogen sulfite, calcium aluminium sulfate and having all degradation impurities less than 1% and 7-epimerized docetaxel less than 0.2% after storage at room temperature for pharmaceutically acceptable duration of time, wherein the degradation impurities are 10-Deacetyl baccatin III, 7-Epi-10-deacetyl baccatin III, 7-Epi-10-oxo-10-deacetyl baccatin III, 7-Epi-docetaxel, 7-Epi-10-oxo-Docetaxel and any individual unspecified unidentified impurity.

According to a further aspect of the invention there is provided a pharmaceutical composition comprising taxane derivative isolated by the process according to the invention.

According to a further aspect of the invention there is provided a process for minimizing the formation of a 7-epimer impurity during isolation of a taxane derivative comprising the steps:
i) forming a reaction mixture of a taxane derivative and a water soluble metal salt selected from the group consisting of aluminum sulfate, potassium aluminum sulfate, lithium biphosphate, sodium dihydrogen phosphate, potassium trisulfite, potassium hydrogen sulfate, potassium dihydrogen phosphate, sodium hydrogen sulfite, calcium aluminium sulfate;
ii) isolating the taxane derivative from the reaction mixture in the presence of the metal salt wherein the degradation product 7 epimer is reduced to less than 0.2% of the isolated taxane derivative.

In a preferred method of the invention said 7-epimer is 7-epi-docetaxel or 7-epi-paclitaxel.

In a preferred method of the invention said taxane derivative is selected from the group consisting of paclitaxel, docetaxel and cabazitaxel.

The present invention provides a process for synthesis of a taxane derivative, preferably Paclitaxel, docetaxel and cabazitaxel either in anhydrous form or in hydrated form, wherein the degradation of the active principal, to its epimer at position 7, which results in the formation of 7-epimer by way of a retro aldol reaction, is controlled and significantly reduced. Thus the product obtained by such a process results in taxane of very high purity. Moreover, taxane derivative obtained by such a process is stable during storage for pharmaceutically acceptable duration of time rendering it highly suitable for commercial purpose.

The present invention provides a process for synthesis of a taxane derivative, wherein the product obtained by such a process withstands the higher temperatures felt during the manufacturing of a pharmaceutical composition. Docetaxel, Paclitaxel or cabazitaxel obtained by the present process can withstand on its own the critical vagaries of temperature, acidic or alkaline environment generated during the manufacturing of pharmaceutical composition.

Thus, the process and the products obtained by the present invention are advantageous with relation to those described in the prioir art, in that they demonstrate superior stability for pharmaceutically acceptable duration of time.

Thus, in one aspect, the present invention provides a simple method to minimize the degradation of taxane derivative either anhydrous or its trihydrate form.

In another aspect, the present invention provides substantially pure docetaxel and paclitaxel free from degradation impurities.

In still another aspect, the present invention provides a substantially pure docetaxel and paclitaxel, which could be stored long enough without undergoing degradation.

In yet another aspect, the present invention provides a method to reduce or minimize the formation of 7-epimer during manufacturing and/or on storage of taxane derivative.

In another aspect the present invention provides a process for minimizing the formation of a 7-epimer impurity during synthesis of a taxane derivative comprising the step of isolating the taxane derivative in the presence of water soluble metal salt.

In still further aspect, the present invention provides pharmaceutical compositions prepared by using Paclitaxel, docetaxel or cabazitaxel prepared according to present invention.

### DETAILED DESCRIPTION OF THE INVENTION

The present invention relates to a method for reducing the degradation impurities formed from taxane derivative either during the reaction or during storage conditions. Thus, taxane derivative obtained by instant process is substantially pure and has relatively more stability when stored for pharmaceutically acceptable duration of time. The present method could be employed to prepare substantially pure anhydrous taxane derivative as well as substantially pure trihydrate taxane derivative.

As used herein the term "substantially pure" means a taxane derivative having all degradation impurities less than 1% and 7-epimerized product less than 0.2% even after storage at room temperature for pharmaceutically acceptable duration of time.

The present inventors while trying to prepare taxane derivatives using prior art methods, observed that such taxane derivatives rapidly undergoes retro aldol reaction giving 7-epimerized product as major degradation product rendering it unsafe for pharmaceutical use.

In order to minimize the degradation of taxane derivative, the present inventors surprisingly found that this could be achieved by carrying isolation of taxane derivative from reaction mixtures in presence of water soluble metal salts. When isolation of taxane derivative, either anhydrous or trihydrate, is carried out in presence of metal salts, it not only renders substantially pure taxane derivative but such a taxane derivative has also good storage stability. The present inventors have observed almost 10-fold reduction in formation of epimerized product when docetaxel isolated in presence of metal salts as compared to those without metal salts, when stored in solution form at room temperature.

As used herein, the term "metal salt" means any molecule having the general formula [M^{q+}]ₐ [X^{z-}]_{b} wherein X is any negatively charged ion, a, b, q and z are independently integers ≥1, q(a)=z(b). The metals salts could be organic or inorganic metal salts. Experiments have shown that if an organic salt is employed, the results are not so good as compared to the utilization of inorganic salts, in terms of storage stability. The only limitation for choice of metal salt is water solubility. The metal salts, which are highly soluble in water, are particularly preferred.

The particularly preferred inorganic metal salts include, but are not limited to, aluminum sulfate, potassium aluminum sulfate, lithium biphosphate, sodium dihydrogen phosphate, potassium trisulfite, potassium hydrogen sulfate, potassium dihydrogen phosphate, sodium hydrogen sulfite, calcium aluminium sulfate. The most preferred metal salt is aluminum sulfate, potassium aluminum sulfate. Further, these metal salts can be added in minimal amounts with concentration from 1.0% to 10% by weight of taxane derivative. Preferably, the metal salts are added in concentration in the range of 1.5-4.0%, and more preferably in the range of 2.0 to 3.0%.

Any synthetic route known in the art could be employed for synthesis of anhydrous as well as for trihydrate form of taxane derivative. Experiments have shown that the stability of the final product is particularly not dependent on the synthetic route employed, if isolation of taxane derivative is carried out in presence of metal salt. According to present invention, irrespective of synthetic routes, if isolation of taxane derivative is carried in presence of metal salts, then the obtained taxane derivative is substantially pure and stable during storage for pharmaceutically acceptable durations. Thus, the use of a metal salt during isolation process of taxane derivative gives the manufacturer the freedom to choose the solvents, reagents, reaction conditions and other parameters. To achieve the stability, according to present invention, it is necessary to isolate the taxane derivative from reaction mixture in presence of metal salts.

The present inventors have studied in detail the impact of addition of aluminium sulfate on formation of epi-docetaxel, the principal degradation product of docetaxel. The results of this study are summarized in Table - 1.

**Table - 1: Comparative stability profile of docetaxel in solution at 35°C with and without Aluminium sulphate**

| **Product** | Without aluminum sulfate (Example - 6) | | | | With aluminum sulfate (Example - 1) | | | |
|---|---|---|---|---|---|---|---|---|
| | Initial | 30min | 60min | 360min | Initial | 30min | 60min | 360min |
| Docetaxel | 99.68% | 99.51% | 98.95% | 98.81% | 99.68% | 99.62% | 99.50% | 99.38 |
| Epi-Docetaxel | 0.11% | 0.23% | 0.34% | 0.86% | 0.11% | 0.11% | 0.12% | 0.19% |

When docetaxel, prepared according to present invention by using aluminium sulphate as metal salts, is compared for formation of principal degradation product, epi-docetaxel, with the one prepared without using aluminium sulphate, the present inventors have observed docetaxel solution at 35°C shows almost five fold reduction. Docetaxel solution prepared using docetaxel according to present invention had 0.19% of epi-docetaxel after 6 hrs whereas other solution showed 0.86% of epi-docetaxel.

The surprising effect of aluminum sulfate during isolation of docetaxel encouraged to evaluate the effect of other metal salt on stability of taxane, in particular docetaxel. The stability data for two months, when docetaxel is synthesized, according to present invention, by using potassium aluminum sulfate, is summarized in Table- 2.

**Table - 2: Comparative stability profile of docetaxel with and without Potassium Aluminium sulphate**

| Related substances by HPLC at 25 ± 2°C / 60 ± 5% RH | With potassium aluminum sulfate (Example - 2) | | | Without potassium aluminum sulfate |
|---|---|---|---|---|
| | Initial | 1 Month | 2 Month | 1 Month |
| 10-Deacetyl baccatin III | ND | 0.01% | 0.01% | 0.07% |
| 7-Epi-10-deacetyl baccatin III | ND | ND | ND | 0.08% |
| 7-Epi-10-oxo-10-deacetyl baccatin III | ND | ND | ND | 0.01% |
| 7-Epi-docetaxel | 0.02% | 0.022% | 0.044% | 0.18% |
| 7-Epi-10-oxo-Docetaxel | ND | ND | ND | 0.01% |
| Any individual unspecified unidentified impurity | 0.07% | 0.076% | 0.083% | 0.07% |
| Total impurities | 0.25% | 0.27% | 0.27% | 0.58% |

| | | | | |
|---|---|---|---|---|
| ND - not detected | | | | |

Thus, typically the present invention provides a method for minimizing the formation of degradation product, especially 7- epimerized product by using metal salts.

In another embodiment, a pharmaceutical formulation is prepared by using taxane derivative prepared according to present invention and polysorbate 80 or cremophore. In particular, a formulation is prepared by using docetaxel isolated in presence of potassium aluminum sulphate and polysorbate 80. A similar formulation is prepared by using polysorbate 80 and docetaxel which was not isolated in presence of any metal salt. The two compositions were kept at 45 °C / 75 % RH for 1-month and stability profile of the formulations was compared.

The results of this study are summarized in following table: 3

**Table 3-Comparison of the formulation with or without isolation of metal salt**

| **No.** | **Formulation** | **Stability (45 0C / 75 % RH)** | **Related substances** | | **Assay (%)** |
|---|---|---|---|---|---|
| | | | **Single Max. Unknown Impurity** | **Total impurities** | |
| 1 | Prepared by using Polysorbate | Initial | 0.736 | 1.99 | 100.8 |
| | 80 and docetaxel (not isolated in presence of any metal salts) | 1 - Month | 1.120 | 3.75 | 88.4 |
| 2 | Prepared by using Polysorbate 80 and docetaxel (isolated in presence of potassium aluminium sulfate) | Initial | 0.043 | 0.14 | 100.1 |
| | | 1 - Month | 0.405 | 0.66 | 99.1 |

From Table 3, it was observed that when docetaxel is isolated in presence of potassium aluminum sulfate, formation of total impurities and single unknown impurity are significantly reduced. Many fold reductions are observed in formation of impurities. Further, when stored at accelerated conditions of storage, assay is reduced only by 0.009% when API is prepared according to present invention, whereas a reduction of 0.124% is observed in assay when docetaxel, which is not isolated in presence of any metal salt, is used.

The invention is further explained with the help of following illustrative examples, however, in no way these examples should be construed as limiting the scope of the invention.

### EXAMPLES

### Example 1

### Preparation of docetaxel trihydrate

The impure 4-acetoxy-2a-benzoyloxy-5β,20-epoxy-1,7β,10β-trihydroxy-9-oxo-tax-11-en-13α-yl (2R,3S)-3-tert-butoxycarbonylamino-2-hydroxy-3-phenylpropionate (30 g) was purified using silica gel flash chromatography utilizing a mixture of Dichloromethane: Methanol in appropriate concentration for elution. The fraction containing the desired product was concentrated under reduced pressure to give a syrupy mass, which was dissolved in a mixture of Ethanol : Cyclohexane : Demineralised water: Ethyl acetate (20:2:10:0.8) containing 0.02g Aluminum sulfate at 25-30°C under constant stirring. The clear biphasic reaction was then concentrated to 20ml and cooled to 5-10°C. Precipitated solid was stirred for 25-30 minutes at 5-10 °C, solid was filtered and washed with DM water (1ml) and dried under reduced pressure under humid atmosphere till moisture content was between 5-7% by Karl Fischer method to give Docetaxel trihydrate in 70-90% yield.

### Example 2

### Preparation of Docetaxel trihydrate

Crude docetaxel (1.0g) was dissolved in a mixture of Ethanol: Cyclohexane: Demineralised water: Ethyl acetate (20:2:10:0.8) containing 0.02g Potassium Aluminum sulfate at 25-30°C under constant stirring. The clear biphasic reaction was then concentrated to 20ml and cooled to 5-10°C. Precipitated solid was stirred for 25-30minutes at 5-10°C, solid was filtered and washed with DM water (1ml) and dried under vacuum under humid atmosphere till moisture content was between 5-7% by Karl Fischer method to give Docetaxel trihydrate in 70-90% yield.

### Example 3

### Preparation of Docetaxel trihydrate

Crude docetaxel (1.0 g,) was dissolved in 1, 4-Dioxane (5ml) at 25-30°C under constant stirring. DM water (7ml) containing 0.02g of Potassium Aluminum sulfate was added slowly at 25-30°C with constant stirring. Precipitated solid was stirred for 25-30 minutes at 25-30°C, solid was filtered, washed with 1:1 solution of 1,4-Dioxane and DM water (1ml) and dried under vacuum under humid atmosphere till moisture content was between 5-7% by Karl Fischer method to give Docetaxel trihydrate in 70-90% yield

### Example 4

### Preparation of Paclitaxel

Crude paclitaxel (1.0g) was dissolved in a mixture of Ethanol: Cyclohexane: Demineralised water: Ethyl acetate (20:2:10:0.8) containing 0.02g Aluminum sulfate at 25-30 °C under constant stirring. The clear biphasic reaction was then concentrated to 20ml and cooled to 5-10°C. Precipitated solid was stirred for 25-30min. at 5-10 °C, solid was filtered and washed with DM water: Ethanol 1:1 mixture (1ml) and dried under vacuum to give Paclitaxel in 70-90% yield.

### Example 5

### Preparation of Paclitaxel

Crude paclitaxel (1.0g) was dissolved in a mixture of Ethanol: Cyclohexane: Demineralised water :Ethyl acetate (20:2:10:0.8) containing 0.02g Potassium Aluminum sulfate at 25-30°C under constant stirring. The clear biphasic reaction was then concentrated to 20ml and cooled to 5-10°C. Precipitated solid was stirred for 25-30min. at 5-10 °C, solid was filtered and washed with DM water: Ethanol 1:1 mixture (1ml) and dried under reduced pressure to give paclitaxel in 70-90% yield.

### Example 6

### Preparation of Docetaxel trihydrate (Comparative Example)

Crude docetaxel (1.0 g,) was dissolved to a solution of Ethanol: Cyclohexane: Demineralised water: Ethyl acetate (20:2:10:0.8) at 25-30°C under constant stirring. The clear biphasic reaction was then concentrated to 20ml and cooled to 5-10°C. Precipitated solid was stirred for 25-30min. at 5-10 °C, solid was filtered and washed with DM water (1ml) and dried under vacuum under humid atmosphere till moisture content is in between 5-7% by Karl Fischer method to give Docetaxel trihydrate in 70-90% yield.

## Claims

1. A process for the preparation of taxane derivatives comprising the steps:
i) forming a reaction mixture of a taxane derivative and a water soluble metal salt selected from the group consisting of aluminum sulfate, potassium aluminum sulfate, lithium biphosphate, sodium dihydrogen phosphate, potassium trisulfite, potassium hydrogen sulfate, potassium dihydrogen phosphate, sodium hydrogen sulfite, calcium aluminium sulfate wherein said salt is provided in a concentration of 1 to 10% by weight of the taxane derivative; and
ii) isolating the taxane derivative from the reaction mixture in the presence of the metal salt wherein the degradation product 7 epimer is reduced to less than 0.2% of the isolated taxane derivative.

2. The process according to claim 1, wherein the metal salt is potassium aluminum sulfate.

3. The process according to claims 1 or 2, wherein the said metal salt is added in concentration of up to 2-3% by weight of taxane derivative.

4. The process according to claim 1 wherein said 7 epimer is 7-epi-docetaxel or 7-epipaclitaxel.

5. Docetaxel isolated in presence of 1-10% of a water soluble metal salt selected from the group consisting of aluminum sulfate, potassium aluminum sulfate, lithium biphosphate, sodium dihydrogen phosphate, potassium trisulfite, potassium hydrogen sulfate, potassium dihydrogen phosphate, sodium hydrogen sulfite, calcium aluminium sulfate and having all degradation impurities less than 1% and 7-epimerized docetaxel less than 0.2% after storage at room temperature for pharmaceutically acceptable duration of time, wherein the degradation impurities are 10-Deacetyl baccatin III, 7-Epi-10-deacetyl baccatin III, 7-Epi-10-oxo-10-deacetyl baccatin III, 7-Epi-docetaxel, 7-Epi-10-oxo-Docetaxel and any individual unspecified unidentified impurity.

6. A pharmaceutical composition comprising taxane derivative isolated according to process of claim 1.

7. A process for minimizing the formation of a 7-epimer impurity during isolation of a taxane derivative comprising the steps:
i) forming a reaction mixture of a taxane derivative and a water soluble metal salt selected from the group consisting of aluminum sulfate, potassium aluminum sulfate, lithium biphosphate, sodium dihydrogen phosphate, potassium trisulfite, potassium hydrogen sulfate, potassium dihydrogen phosphate, sodium hydrogen sulfite, calcium aluminium sulfate;
ii) isolating the taxane derivative from the reaction mixture in the presence of the metal salt wherein the degradation product 7 epimer is reduced to less than 0.2% of the isolated taxane derivative.

8. The process according to claim 7 wherein said 7-epimer is 7-epi-docetaxel or 7-epipaclitaxel.

9. The process according to claim any one of claims7 or 8, wherein the taxane derivative is selected from the group consisting of paclitaxel, docetaxel and cabazitaxel.

## Patentansprüche

1. Verfahren zur Herstellung von Taxanderivaten, umfassend die Schritte:
i) Bilden einer Reaktionsmischung eines Taxanderivats und eines wasserlöslichen Metallsalzes ausgewählt aus der Gruppe bestehend aus Aluminiumsulfat, Kaliumaluminiumsulfat, Lithiumbiphosphat, Natriumdihydrogenphosphat, Kaliumtrisulfit, Kaliumhydrogensulfat, Kaliumdihydrogenphosphat, Natriumhydrogensulfit, Calciumaluminiumsulfat, wobei das Salz in einer Konzentration von 1 bis 10 Gew.-% des Taxanderivats bereitgestellt wird; und
ii) Isolieren des Taxanderivats aus der Reaktionsmischung in Gegenwart des Metallsalzes, wobei das Abbauprodukt 7-Epimer auf weniger als 0,2 % des isolierten Taxanderivats reduziert wird.

2. Verfahren nach Anspruch 1, wobei das Metallsalz Kaliumaluminiumsulfat ist.

3. Verfahren nach den Ansprüchen 1 oder 2, wobei das Metallsalz in einer Konzentration von bis zu 2-3 Gew.-% des Taxanderivats zugefügt wird.

4. Verfahren nach Anspruch 1, wobei das 7-Epimer 7-Epi-docetaxel oder 7-Epi-paclitaxel ist.

5. Docetaxel, isoliert in Gegenwart von 1-10 % eines wasserlöslichen Metallsalzes ausgewählt aus der Gruppe bestehend aus Aluminiumsulfat, Kaliumaluminiumsulfat, Lithiumbiphosphat, Natriumdihydrogenphosphat, Kaliumtrisulfit, Kaliumhydrogensulfat, Kaliumdihydrogenphosphat, Natriumhydrogensulfit, Calciumaluminiumsulfat, und wobei nach Lagerung bei Raumtemperatur für eine pharmazeutisch annehmbare Zeitdauer alle Verunreinigungen durch Abbau weniger als 1 % betragen und 7-epimerisiertes Docetaxel weniger als 0,2 % beträgt, wobei die Verunreinigungen durch Abbau 10-Deacetylbaccatin III, 7- Epi-10-deacetylbaccatin III, 7-Epi-10-oxo-10-deacetylbaccatin III, 7-Epi-docetaxel, 7-Epi-10-oxo-docetaxel und jegliche individuelle unspezifizierte unidentifizierte Verunreinigung sind.

6. Pharmazeutische Zusammensetzung, umfassend Taxanderivat, das gemäß dem Verfahren von Anspruch 1 isoliert wird.

7. Verfahren zum Minimieren der Bildung einer 7-Epimerverunreinigung während der Isolierung eines Taxanderivats, umfassend die Schritte:
i) Bilden einer Reaktionsmischung eines Taxanderivats und eines wasserlöslichen Metallsalzes ausgewählt aus der Gruppe bestehend aus Aluminiumsulfat, Kaliumaluminiumsulfat, Lithiumbiphosphat, Natriumdihydrogenphosphat, Kaliumtrisulfit, Kaliumhydrogensulfat, Kaliumdihydrogenphosphat, Natriumhydrogensulfit, Calciumaluminiumsulfat;
ii) Isolieren des Taxanderivats aus der Reaktionsmischung in Gegenwart des Metallsalzes, wobei das Abbauprodukt 7-Epimer auf weniger als 0,2 % des isolierten Taxanderivats reduziert wird.

8. Verfahren nach Anspruch 7, wobei das 7-Epimer 7-Epi-docetaxel oder 7-Epi-paclitaxel ist.

9. Verfahren nach einem der Ansprüche 7 oder 8, wobei das Taxanderivat ausgewählt ist aus der Gruppe bestehend aus Paclitaxel, Docetaxel und Cabazitaxel.

## Revendications

1. Procédé pour la préparation de dérivés de taxane comprenant les étapes consistant à :
i) former un mélange réactionnel d'un dérivé de taxane et d'un sel métallique soluble dans l'eau choisi dans le groupe constitué du sulfate d'aluminium, du sulfate d'aluminium et de potassium, du diphosphate de lithium, du dihydrogénophosphate de sodium, du trisulfite de potassium, de l'hydrogénosulfate de potassium, du dihydrogénophosphate de potassium, de l'hydrogénosulfite de sodium, du sulfate d'aluminium et de calcium, dans lequel ledit sel est fourni à une concentration de 1 à 10 % en poids du dérivé de taxane ; et à
(ii) isoler le dérivé de taxane du mélange réactionnel en présence du sel métallique dans lequel le produit de dégradation épimère 7 est réduit à moins de 0,2 % du dérivé de taxane isolé.

2. Procédé selon la revendication 1, dans lequel le sel métallique est le sulfate d'aluminium et de potassium.

3. Procédé selon les revendications 1 ou 2, dans lequel ledit sel métallique est ajouté à une concentration allant jusqu'à 2 à 3 % en poids du dérivé de taxane.

4. Procédé selon la revendication 1, dans lequel ledit épimère 7 est le 7-épi-docétaxel ou le 7-épi-paclitaxel.

5. Docétaxel isolé en présence de 1 à 10 % d'un sel métallique soluble dans l'eau choisi dans le groupe constitué du sulfate d'aluminium, du sulfate d'aluminium et de potassium, du diphosphate de lithium, du dihydrogénophosphate de sodium, du trisulfite de potassium, de l'hydrogénosulfate de potassium, du dihydrogénophosphate de potassium, de l'hydrogénosulfite de sodium, du sulfate d'aluminium et de calcium et ayant toutes les impuretés de dégradation inférieures à 1 % et le docétaxel 7-épimérisé inférieur à 0,2 % après conservation à température ambiante pendant un laps de temps pharmaceutiquement acceptable, dans lequel les impuretés de dégradation sont la 10-désacétylbaccatine III, la 7-épi-10-désacétylbaccatine III, la 7-épi-10-oxo-10-désacétylbaccatine III, le 7-épi-docétaxel, le 7-épi-10-oxo-docétaxel et n'importe quelle impureté individuelle non identifiée et non spécifiée.

6. Composition pharmaceutique comprenant un dérivé de taxane isolé selon le procédé de la revendication 1.

7. Procédé pour minimiser la formation d'une impureté épimère 7 durant l'isolation d'un dérivé de taxane comprenant les étapes consistant à :
i) former un mélange réactionnel d'un dérivé de taxane et d'un sel métallique soluble dans l'eau choisi dans le groupe constitué du sulfate d'aluminium, du sulfate d'aluminium et de potassium, du diphosphate de lithium, du dihydrogénophosphate de sodium, du trisulfite de potassium, de l'hydrogénosulfate de potassium, du dihydrogénophosphate de potassium, de l'hydrogénosulfite de sodium, du sulfate d'aluminium et de calcium ;
(ii) isoler le dérivé de taxane du mélange réactionnel en présence du sel métallique dans lequel le produit de dégradation épimère 7 est réduit à moins de 0,2 % du dérivé de taxane isolé.

8. Procédé selon la revendication 7, dans lequel ledit épimère 7 est le 7-épi-docétaxel ou le 7-épi-paclitaxel.

9. Procédé selon l'une quelconque des revendications 7 ou 8, dans lequel le dérivé de taxane est choisi dans le groupe constitué du paclitaxel, du docétaxel et du cabazitaxel.
